(19) Europäisches Patentamt
European Patent Office
Office européen des brevets

(11) Veröffentlichungsnummer : **0 091 051**
**B1**

(12) # EUROPÄISCHE PATENTSCHRIFT

(45) Veröffentlichungstag der Patentschrift : **03.12.86**

(51) Int. Cl.⁴ : **C 07 D335/02**, C 07 D495/04, A 61 K 31/38

(21) Anmeldenummer : **83103043.2**

(22) Anmeldetag : **28.03.83**

(54) **4H-Thiopyrane, Verfahren zu ihrer Herstellung und ihre Verwendung in Arzneimitteln.**

(30) Priorität : **06.04.82 DE 3212737**

(43) Veröffentlichungstag der Anmeldung : **12.10.83 Patentblatt 83/41**

(45) Bekanntmachung des Hinweises auf die Patenterteilung : **03.12.86 Patentblatt 86/49**

(84) Benannte Vertragsstaaten : **AT BE CH DE FR GB IT LI NL SE**

(56) Entgegenhaltungen :
**FR-A- 2 310 128**
**US-A- 4 198 426**
**TETRAHEDRON, Band 37, Nr. 21, 1981, London J.B. RASMUSSEN et al. "Enamine chemistry - XXIII. The [4+2] cycloaddition reactions between enaminothiones and electrophilic olefins and acetylenes", pages 3693-3698**
**Chemical Abstracts Band 93, Nr. 23, 8. Dezember 1980, Columbus, Ohio, USA W. RIED et al. "Synthesis and reactivity of 4H-dithieno[2,3-b:3',2'-e]thiopyran derivatives", Seite 519, Spalte 2, Abstract Nr. 220625t**
**Chemical Abstracts Band 71, Nr. 20, 17. November 1969, Columbus, Ohio, USA A.M. ARISHKEVICH et al. "2,6-Dimercapto-4H-thiopyran-4-ones as new titrants for the amperometric determination of selenium(IV)", Seite 560, Spalte 1, Abstract Nr. 98032A**
**J. Heterocycl. Chem. 17, 405 (1980), Tetrahedron 23, 3785 (1967)**
**J. AM. Chem. 91, 208 (1969), Tetrahedron Ltr. 1971, 2241**
**Die Akte enthält technische Angaben, die nach dem Eingang der Anmeldung eingereicht wurden und die nicht in dieser Patentschrift enthalten sind.**

(73) Patentinhaber : **BAYER AG**
**Konzernverwaltung RP Patentabteilung**
**D-5090 Leverkusen 1 Bayerwerk (DE)**

(72) Erfinder : **Goldmann, Siegfried, Dr.**
**Kuckuckstrasse 41**
**D-5600 Wuppertal 2 (DE)**
Erfinder : **Thomas, Günter, Dr.**
**Claudiusweg 9**
**D-5600 Wuppertal 1 (DE)**
Erfinder : **Schramm, Matthias, Dr.**
**Paffrather Strasse 38**
**D-5000 Köln 80 (DE)**

**EP 0 091 051 B1**

Anmerkung : Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Jouve, 18, rue St-Denis, 75001 Paris, France

**Beschreibung**

Die vorliegende Erfindung betrifft 4H-Thiopyrane, ein Verfahren zu ihrer Herstellung sowie ihre Verwendung in Arzneimitteln, insbesondere in kreislaufbeeinflussenden Mitteln.

4H-Thiopyrane und deren Herstellung durch Umsetzen von Dimethylacetylencarboxylat mit Enaminthionen sind von Rasmussen et al. (Tetrahedron *37*, 3693 (1981)) beschrieben worden. Auch Nishio et al. (J. Heterocycl. Chem. *17*, 405 (1980)), Smutny, Turner (Tetrahedron *23*, 3785 (1967)), Smutny (J. Am. Chem. Soc. *91*, 208 (1969)) und Kalish (Tetrahedron Lett. *1971*, 2241)) haben gezeigt, daß α,β-ungesättigte Thione bzw. Dithioester zu Diels-Alder-Reaktionen befähigt sind.

Bisher nicht bekannt sind Diels-Alder-Reaktionen von α,β-ungesättigten Thioestern, die keine ionischen mesomeren Grenzformen bilden können, (Darstellung : vgl. Scheithauer, Mayer, Chem. Ber. *100*, 1413 (1967), Barnikow, Strickmann, Chem. Ber. *100*, 1428 (1967) mit Acetylenen, die zu 2-Alkoxy-Thiopyranen führen würden.

Die vorliegende Erfindung betrifft 4H-Thiopyrane der allgemeinen Formel (I),

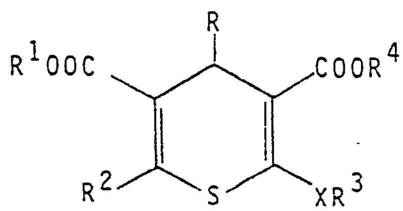

(I)

in welcher

R für Phenyl, Naphthyl, Furyl, Pyrryl oder Pyridyl steht, wobei diese Ringe gegebenenfalls 1, 2 oder 3 gleiche oder verschiedene Substituenten aus der Gruppe Alkyl, Alkenyl, Alkinyl, Alkoxy, Alkylen, Alkylendioxy, Alkylamino mit jeweils bis zu 4 Kohlenstoffatomen, Phenyl, Halogen, Trifluormethyl, Trifluormethoxy, Nitro, Cyano oder Azido enthalten,

$R^1$, $R^3$ und $R^4$ gleich oder verschieden sind und jeweils für Wasserstoff, einen Alkylrest mit bis zu 6 Kohlenstoffatomen oder für einen der unter R genannten Reste stehen,

wobei $R^4$ außerdem eine Einfachbindung zu R unter Bildung eines 6- bis 7-gliedrigen Lactonringes darstellen kann ;

$R^2$ für Wasserstoff, einen Alkylrest mit bis zu 4 Kohlenstoffatomen, Phenyl oder Aminoalkyl mit bis zu 4 Kohlenstoffatomen steht, und

X für O, S oder —NH— steht,

sowie ihre pharmazeutisch unbedenklichen Säureadditionssalze.

Bevorzugt sind Verbindungen der allgemeinen Formel I, in welcher

R für Phenyl, Naphthyl oder Pyridyl steht, wobei diese Ringe gegebenenfalls 1 oder 2 gleiche oder verschiedene Substituenten aus der Gruppe Alkyl, Alkoxy, Alkylen, Alkylendioxy, Alkylamino mit jeweils bis zu 4 Kohlenstoffatomen, Phenyl, Halogen, Trifluormethyl, Trifluormethoxy, Nitro, Cyano oder Azido enthalten,

$R^1$, $R^3$ und $R^4$ gleich oder verschieden sind und jeweils für Wasserstoff, einen Alkylrest mit bis zu 4 Kohlenstoffatomen oder Phenyl stehen, wobei $R^4$ außerdem eine Einfachbindung zu R unter Bildung eines 6- bis 7-gliedrigen Lactonringes darstellen kann,

$R^2$ für Wasserstoff, einen Alkylrest mit bis zu 4 Kohlenstoffatomen, Phenyl oder Aminoalkyl mit bis zu 4 Kohlenstoffatomen steht, und

X für O, S oder —NH— steht,

sowie ihre pharmazeutisch unbedenklichen Säureadditionssalze.

Die Erfindung betrifft weiterhin ein Verfahren zur Herstellung von Verbindungen der allgemeinen Formel (I), welches dadurch gekennzeichnet ist, daß man Thioverbindungen der allgemeinen Formel (II)

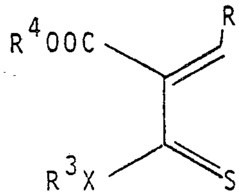

(II)

in welcher R, $R^3$, $R^4$ und X die oben angegebene Bedeutung haben, bei Temperaturen zwischen 0 °C und 200 °C, gegebenenfalls in Gegenwart von inerten organischen Lösungsmitteln, mit Acetylenverbindungen der allgemeinen Formel (III),

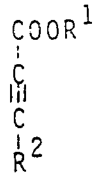

(III)

in der $R^1$ und $R^2$ die oben angegebenen Bedeutungen haben, umsetzt.

Verwendet man Benzyliden-thiomalonsäuremethylester und Acetylencarbonsäuremethylester, so kann der Reaktionsverlauf durch das folgende Formelschema wiedergegeben werden :

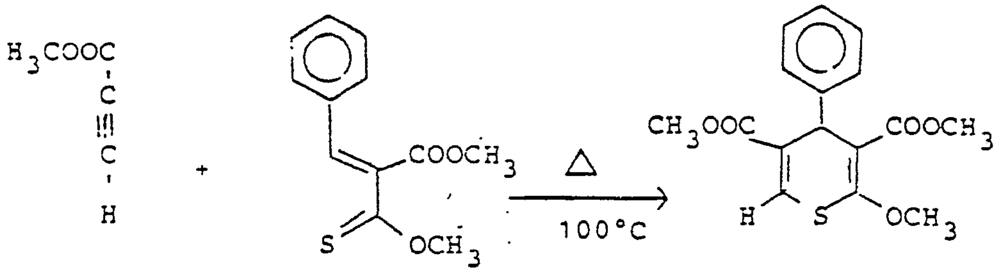


Die als Ausgangsstoffe verwendeten Benzyliden-thiomalonsäureester der allgemeinen Formel (II) sind bekannt oder können nach bekannten Methoden dargestellt werden (Scheithauer und Mayer, Chem. Ber. *100*, 1413 (1967), Barnikow, Strickmann, Chem. Ber. *100*, 1428 (1967)).

Die als Ausgangsstoffe verwendeten Acetylencarbonsäureester der allgemeinen Formel (III) sind ebenfalls bekannt oder können nach bekannten Verfahren dargestellt werden.

Als Lösungsmittel können die im Überschuß eingesetzten Acetylencarbonsäureester der Formel (III) dienen oder aber inerte organische Lösungsmittel wie Toluol, Ethanol, Dioxan oder Ethylenglycol.

Bei der Durchführung des erfindungsgemäßen Verfahrens arbeitet man vorzugsweise in einem Temperaturbereich von 0 ° bis 200 °C, insbesondere in einem Bereich von 50° bis 150 °C.

Die Umsetzung erfolgt üblicherweise bei Normaldruck, kann aber auch bei erhöhtem Druck durchgeführt werden.

Die erfindungsgemäßen neuen Thiopyrane der allgemeinen Formel (I) besitzen wertvolle pharmakologische Eigenschaften. Aufgrund ihrer kreislaufbeeinflussenden Wirkung können sie als Vasodilatatoren sowie als Coronartherapeutika Verwendung finden und sind somit als Bereicherung der Pharmazie anzusehen.

Die erfindungsgemäßen Verbindungen zeigen interessante biologische Wirkungen. Sie besitzen ein breites und vielseitiges pharmakologisches Wirkungsspektrum. Im einzelnen seien folgende Hauptwirkungen genannt :

1. Die Verbindungen bewirken bei parenteraler, oraler und perlingualer Zugabe eine deutliche und langanhaltende Erweiterung der Coronargefäße. Diese Wirkung auf die Coronargefäße wird durch einen gleichzeitigen nitritähnlichen herzentlastenden Effekt verstärkt.

Sie beeinflussen bzw. verändern den Herzstoffwechsel im Sinne einer Energieersparnis.

2. Die Erregbarkeit des Reizbildungs- und Erregungsleitungssystems innerhalb des Herzens wird herabgesetzt, so daß eine in therapeutischen Dosen nachweisbare Antiflimmerwirkung resultiert.

3. Der Tonus der glatten Muskulatur der Gefäße wird unter der Wirkung der Verbindungen stark vermindert. Diese gefäßspasmolytische Wirkung kann im gesamten Gefäßsystem stattfinden, oder sich mehr oder weniger isoliert in umschriebenen Gefäßgebieten (wie z. B. dem Zentralnervensystem) manifestieren.

4. Die Verbindungen senken den Blutdruck von normotonen und hypertonen Tieren und können somit als antihypertensive Mittel verwendet werden.

5. Die Verbindungen sind positiv inotrop und können somit als Kardiotonika Verwendung finden.

6. Die Verbindungen haben stark muskulär-spasmolytische Wirkungen, die an der glatten Muskulatur des Magens, Darmtraktes, des Urogenitaltraktes und des Respirationssystems deutlich werden.

Die neuen Wirkstoffe können in bekannter Weise in die üblichen Formulierungen überführt werden, wie Tabletten, Kapseln, Dragees, Pillen, Granulate, Aerosole, Sirupe, Emulsionen, Suspensionen und Lösungen, unter Verwendung inerter, nicht-toxischer, pharmazeutisch geeigneter Trägerstoffe oder Lösungsmittel. Hierbei soll die therapeutische wirksame Verbindung jeweils in einer Konzentration von etwa 0,5 bis 90 Gew.-% der Gesamtmischung vorhanden sein, d. h. in Mengen, die ausreichend sind, um den angegebenen Dosierungsspielraum zu erreichen.

Die Formulierungen werden beispielsweise hergestellt durch Verstrecken der Wirkstoffe mit Lösungsmitteln und/oder Trägerstoffen, gegebenenfalls unter Verwendung von Emulgiermitteln und/oder Dispergiermitteln, wobei z. B. im Fall der Benutzung von Wasser als Verdünnungsmittel gegebenenfalls organische Lösungsmittel als Hilfslösungsmittel verwendet werden können.

Als Hilfsstoffe seien beispielsweise aufgeführt :

Wasser, nicht-toxische organische Lösungsmittel, wie Paraffine (z. B. Erdolfraktionen), pflanzliche

Öle (z. B. Erdnuß-/Sesamöl), Alkohole (z. B. Ethylalkohol, Glycerin), Glykole (z. B. Propylenglykol, Polyethylenglykol), feste Trägerstoffe, wie z. B. natürliche Gesteinsmehle (z. B. Kaoline, Tonerden, Talkum, Kreide), synthetische Gesteinsmehle (z. B. hochdisperse Kieselsäure, Silikate), Zucker (z. B. Rohr-, Milch- und Traubenzucker), Emulgiermittel (z. B. Polyoxyethylen-Fettsäure-Ester, Polyoxyethylen-Fettalkohol-Ether, Alkylsulfonate und Arylsulfonate), Dispergiermittel (z. B. Lignin, Sulfitablaugen, Methylcellulose, Stärke und Polyvinylpyrrolidon) und Gleitmittel (z. B. Magnesiumstearat, Talkum, Stearinsäure und Natriumlaurylsulfat).

Die Applikation erfolgt in üblicher Weise, vorzugsweise oral oder parenteral, insbesondere perlingual oder intravenös. Im Falle der oralen Anwendung können Tabletten selbstverständlich außer den genannten Trägerstoffen auch Zusätze, wie Natriumcitrat, Calciumcarbonat und Dicalciumphosphat zusammen mit verschiedenen Zuschlagstoffen, wie Stärke, vorzugsweise Kartoffelstärke, Gelatine und dergleichen enthalten. Weiterhin können Gleitmittel, wie Magnesiumstearat, Natriumlaurylsulfat und Talkum zum Tablettieren mitverwendet werden. Im Falle wäßriger Suspensionen und/oder Elixiere, die für orale Anwendungen gedacht sind, können die Wirkstoffe außer den oben genannten Hilfsstoffen mit verschiedenen Geschmacksaufbesserern oder Farbstoffen versetzt werden.

Für den Fall der parenteralen Anwendung können Lösungen der Wirkstoffe unter Verwendung geeigneter flüssiger Trägermaterialien eingesetzt werden.

Im allgemeinen hat es sich als vorteilhaft erwiesen, bei intravenöser Applikation Mengen von etwa 0,01 bis 10 mg/kg, vorzugsweise etwa 0,05 bis 5 mg/kg Körpergewicht pro Tag zur Erzielung wirksamer Ergebnisse zu verabreichen. Bei oraler Applikation beträgt die Dosierung etwa 0,05 bis 20 mg/kg, vorzugsweise 0,5 bis 5 mg/kg Körpergewicht pro Tag.

Trotzdem kann es gegebenenfalls erforderlich sein, von den genannten Mengen abzuweichen, und zwar in Abhängigkeit vom Körpergewicht des Versuchstieres bzw. der Art des Applikationsweges, aber auch aufgrund der Tierart und deren individuellem Verhalten gegenüber dem Medikament bzw. deren Art, von dessen Formulierung und dem Zeitpunkt bzw. dem Intervall, zu welchem die Verabreichung erfolgt. So kann es in einigen Fällen ausreichend sein, mit weniger als der vorgenannten Mindestmenge auszukommen, während in anderen Fällen die genannte obere Grenze überschritten werden muß. Im Falle der Applikation größerer Mengen kann es empfehlenswert sein, diese in mehrere Einzelgaben über den Tag zu verteilen. Für die Applikation in der Humanmedizin ist der gleiche Dosierungsspielraum vorgesehen. Sinngemäß gelten hierbei auch die obigen Ausführungen.

## Beispiel 1

4-(2,4-Dichlorphenyl)-2-methoxy-4H-thiopyran-3,5-dicarbonsäuredimethylester

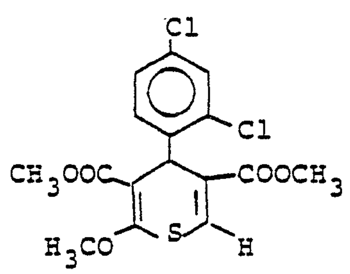

3,1 g (10 mmol) 2,4-Dichlorbenzyliden-thiomalonsäuredimethylester wurden mit 6 ml Propiolsäuremethylester 3 h am Rückfluß erhitzt. Alles Flüchtige wurde im Vakuum abgezogen. Man erhielt 2,7 g (70 %) als Öl.

$^1H$—NMR ($CDCl_3$) : δ 3.6 (s, 6H), 3.9 (s, 3H), 5.6 (s, 1H), 7.1-7.6 (m, 4H)

Analog Beispiel 1 wurden hergestellt :

## Beispiel 2

4-(4-Nitrophenyl)-2-ethoxy-4H-thiopyran-3,5-dicarbonsäure-diethylester

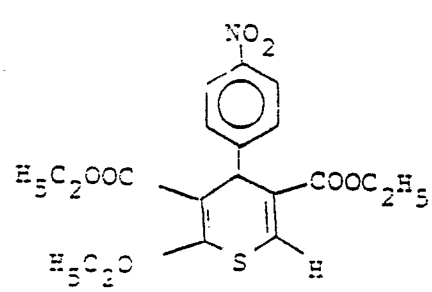

Aus 4-Nitrobenzyliden-thiomalonsäure-diethylester und Propiolsäureethylester gelöst in 5 ml Toluol.
$^1H$—NMR ($CDCl_3$) : δ = 1.25 (t, J = 7Hz, 6H), 1.4 (t, J = 7Hz, 3H), 4.2 (q, J = 7Hz, 6H), 5.5 (s, 1H), 7.6 (s, 1H), 7.6 (d, J = 9Hz, 2H), 8.1 (d, J = 9Hz, 2H).
MS : 407 ($M^+$, 11 %), 334 (100 %), 211 (80 %).
**Ausbeute : 90 %**

Beispiel 3

4-(3-Nitrophenyl)-2-ethoxy-4H-thiopyran-3,5-dicarbonsäure-diethylester

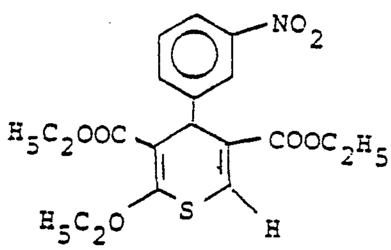

Aus 3-Nitrobenzyliden-thiomalonsäure-diethylester und Propiolsäureethylester
$^1H$—NMR ($CDCl_3$) : δ = 1.3 (t, J = 8Hz, 6H), 1.45 (t, J = 8Hz, 3H), 4.1-4.4 (m, 6H), 5.5 (s, 1H), 7.5 (t, J = 7Hz, 1H), 7.65 (s, 1H), 7.85 (d, J = 7Hz, 1H), 8.1 (d, J = 7Hz, 1H), 8.35 (s, 1H).
Ausbeute : 40 % (nach Chromatographie mit $CHCl_3$ an Kieselgel).

Beispiel 4

4-(2-Trifluormethyl-phenyl)-2-ethoxy-4H-thiopyran-3,5-dicarbonsäure-diethylester

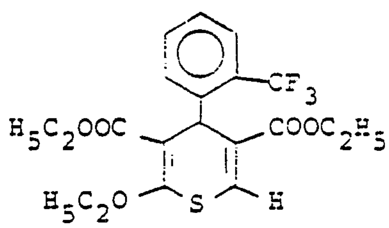

$^1H$—NMR ($CDCl_3$) : δ = 1.1-1.6 (m, 9H), 3.9-4.5 (m, 6H), 5.8 (s, 1H), 7.3-8.1 (m, 5H).
Ausbeute : 75 %

Beispiel 5

4-(2-Chlorphenyl)-2-methoxy-4H-thiopyran-3,5-dicarbonsäure-dimethylester

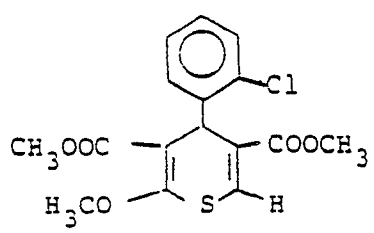

$^1H$—NMR ($CDCl_3$) : δ = 3.65 (s, 6H), 3.8 (s, 3H), 5.65 (s, 1H), 7.0-7.6 (m, 4H), 7.55 (s, 1H).
MS : 354 ($M^+$, 2 %), 295 (7 %), 235 (100 %).
Ausbeute : 77 %

Beispiel 6

4-(3-Chlorphenyl)-2-methoxy-4H-thiopyran-3,5-dicarbonsäure-dimethylester

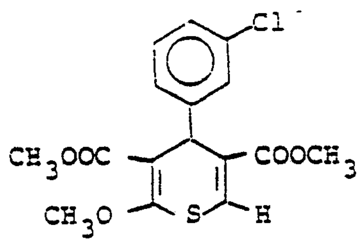

Fp : 85-87°
Ausbeute : 43 %

Beispiel 7

4-Methoxy-9-nitro-5-oxo-5H,10bH-thiopyrano [3,4-c]-l-benzopyran-1-carbonsäure-methylester

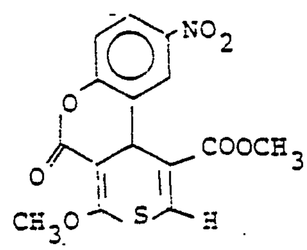

Ausbeute 2,2 g (60 %), Fp : 230 °C (aus Essigester).

Beispiel 8

4-Ethoxy-5-oxo-5H,10bH-thiopyrano [3,4-c]-l-benzopyran-1-carbonsäure-ethylester

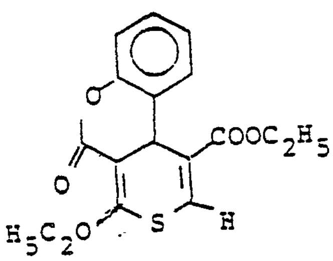

Fp : 135-136 °C (aus Aceton)
Ausbeute : 80 %

Beispiel 9

4-Ethoxy-9-nitro-5-oxo-5H,10bH-thiopyrano [3,4-c]-l-benzopyran-1-carbonsäure-ethylester

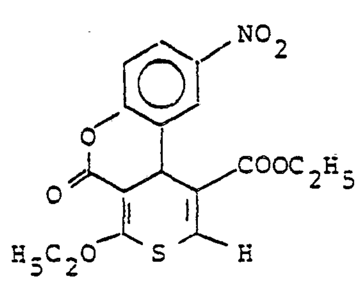

Fp : 131-133 °C (aus Ethanol)
Ausbeute : 67 %

Beispiel 10

9-Brom-4-ethoxy-5-oxo-5H,10bH-thiopyrano [3,4-c]-l-benzopyran-1-carbonsäure-ethylester

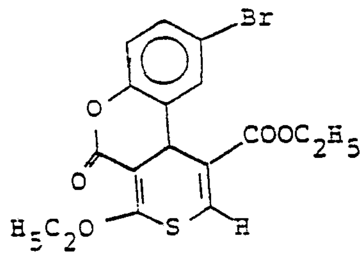

$^1H$—NMR ($CDCl_3$) : δ = 1.3 und 1.35 (2t, J = 7Hz, je 3H), 4.1 und 4.3 (2q, J = 7Hz, je 2H), 5.1 (s, 1H), 6.8-7.8 (m, 3H), 7.6 (s, 1H).

Ausbeute : 74 %

Beispiel 11

7,9-Dichlor-4-ethoxy-5-oxo-5H,10bH-thiopyrano [3,4-c]-I-benzopyran-1-carbonsäure-ethylester

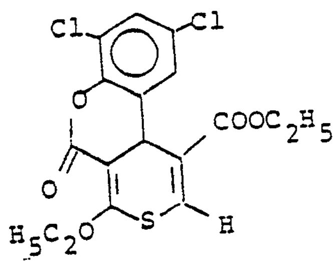

Fp : 148-149 °C
Ausbeute : 86 %

Beispiel 12

7,9-Dichlor-4-methoxy-5-oxo-5H,10bH-thiopyrano [3,4-c]-I-benzopyran-1-carbonsäure-methylester

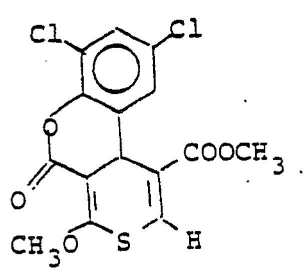

Fp : 202-203 °C (aus Essigester)
Ausbeute : 56 %

Beispiel 13

4-Ethoxy-9-nitro-5-oxo-5H, 10bH-thiopyrano [3,4-c]-I-benzopyran-1-carbonsäure-methylester

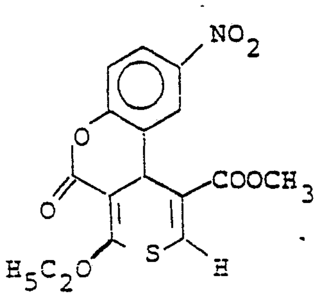

Fp : 156 °C
Ausbeute : 63 %

**Patentansprüche**

1. Thiopyrane der allgemeinen Formel (I)

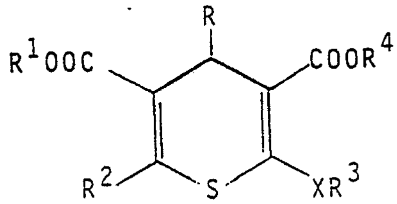

(I)

in welcher

R für Phenyl, Naphthyl, Furyl, Pyrryl oder Pyridyl steht, wobei diese Ringe gegebenenfalls 1, 2 oder 3 gleiche oder verschiedene Substituenten aus der Gruppe Alkyl, Alkenyl, Alkinyl, Alkoxy, Alkylen, Alkylendioxy, Alkylamino mit jeweils bis zu 4 Kohlenstoffatomen, Phenyl, Halogen, Trifluormethyl, Trifluormethoxy, Nitro, Cyano oder Azido enthalten,

$R^1$, $R^3$ und $R^4$ gleich oder verschieden sind und jeweils für Wasserstoff, einen Alkylrest mit bis zu 6 Kohlenstoffatomen oder für einen der unter R genannten Reste stehen, wobei $R^4$ außerdem eine Einfachbindung zu R unter Bildung eines 6- bis 7-gliedrigen Lactonringes darstellen kann ;

$R^2$ für Wasserstoff, einen Alkylrest mit bis zu 4 Kohlenstoffatomen, Phenyl oder Aminoalkyl mit bis zu 4 Kohlenstoffatomen steht, und

X für O, S oder —NH— steht,

sowie ihre pharmazeutisch unbedenklichen Säureadditionssalze.

2. Thiopyrane gemäß Anspruch 1, dadurch gekennzeichnet, daß in der allgemeinen Formel (I)

R für Phenyl, Naphthyl oder Pyridyl steht, wobei diese Ringe gegebenenfalls 1 oder 2 gleiche oder verschiedene Substituenten aus der Gruppe Alkyl, Alkoxy, Alkylen, Alkylendioxy, Alkylamino mit jeweils bis zu 4 Kohlenstoffatomen, Phenyl, Halogen, Trifluormethyl, Trifluormethoxy, Nitro, Cyano oder Azido enthalten,

$R^1$, $R^3$ und $R^4$ gleich oder verschieden sind und jeweils für Wasserstoff, einen Alkylrest mit bis zu 4 Kohlenstoffatomen oder Phenyl stehen, wobei $R^4$ außerdem eine Einfachbindung zur R unter Bildung eines 6- bis 7-gliedrigen Lactonringes darstellen kann,

$R^2$ für Wasserstoff, einen Alkylrest mit bis zu 4 Kohlenstoffatomen, Phenyl oder Aminoalkyl mit bis zu 4 Kohlenstoffatomen steht, und

X für O, S oder —NH— steht,

sowie ihre pharmazeutisch unbedenklichen Säureadditionssalze.

3. Verfahren zur Herstellung von Thiopyranen der allgemeinen Formel (I)

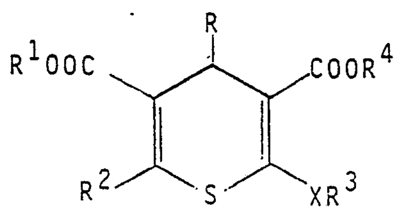

(I)

in welcher

R für Phenyl, Naphthyl, Furyl, Pyrryl oder Pyridyl steht, wobei diese Ringe gegebenenfalls 1, 2 oder 3 gleiche oder verschiedene Substituenten aus der Gruppe Alkyl, Alkenyl, Alkinyl, Alkoxy, Alkylen, Alkylendioxy, Alkylamino mit jeweils bis zu 4 Kohlenstoffatomen, Phenyl, Halogen, Trifluormethyl, Trifluormethoxy, Nitro, Cyano oder Azido enthalten,

$R^1$, $R^3$ und $R^4$ gleich oder verschieden sind und jeweils für Wasserstoff, einen Alkylrest mit bis zu 6 Kohlenstoffatomen oder für einen der unter R genannten Reste stehen, wobei $R^4$ außerdem eine Einfachbindung zu R unter Bildung eines 6- bis 7-gliedrigen Lactonringes darstellen kann ;

$R^2$ für Wasserstoff, einen Alkylrest mit bis zu 4 Kohlenstoffatomen, Phenyl oder Aminoalkyl mit bis zu 4 Kohlenstoffatomen steht, und

X für O, S oder —NH— steht,

sowie ihren pharmazeutisch unbedenklichen Säureadditionssalzen, dadurch gekennzeichnet, daß man Thioverbindungen der allgemeinen Formel (II)

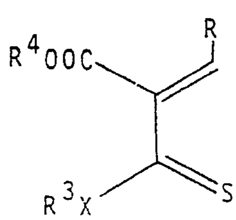

(II)

in welcher R, $R^3$, $R^4$ und X die oben angegebenen Bedeutungen haben, bei Temperaturen zwischen 0 °C und 200 °C, gegebenenfalls in Gegenwart von inerten organischen Lösungsmitteln, mit Acetylenverbindungen der allgemeinen Formel (III)

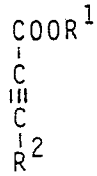

(III)

in der $R^1$ und $R^2$ die oben angegebenen Bedeutungen haben, umsetzt.

4. Verfahren gemäß Anspruch 3 zur Herstellung von Thiopyranen der allgemeinen Formel (I), in welcher

R für Phenyl, Naphthyl, Furyl, Pyrryl oder Pyridyl steht, wobei diese Ringe gegebenenfalls 1, 2 oder 3 gleiche oder verschiedene Substituenten aus der Gruppe Alkyl, Alkoxy, Alkylen, Alkylendioxy, Alkylamino mit jeweils bis zu 4 Kohlenstoffatomen, Phenyl, Halogen, Trifluormethyl, Trifluormethoxy, Nitro, Cyano oder Azido enthalten,

$R^1$, $R^3$ und $R^4$ gleich oder verschieden sind und jeweils für Wasserstoff, einen Alkylrest mit bis zu 4 Kohlenstoffatomen oder für einen der unter R genannten Reste stehen, wobei $R^4$ außerdem eine Einfachbindung zu R unter Bildung eines 6- bis 7-gliedrigen Lactonringes darstellen kann ;

$R^2$ für Wasserstoff, einen Alkylrest mit bis zu 4 Kohlenstoffatomen, Phenyl oder Aminoalkyl mit bis zu 4 Kohlenstoffatomen steht, und

X für O, S oder —NH— steht,

sowie ihren pharmazeutisch unbedenklichen Säureadditionssalzen.

5. Verfahren gemäß den Ansprüchen 3 oder 4, dadurch gekennzeichnet, daß man die Umsetzung bei Temperaturen zwischen 50 und 150 °C durchführt.

6. Verfahren gemäß den Ansprüchen 3 oder 4, dadurch gekennzeichnet, daß man die Umsetzung in einem Überschuß der eingesetzten Acetylencarbonsäureester oder in einem inerten organischen Lösungsmittel wie Toluol, Ethanol, Dioxan oder Ethylenglykol durchführt.

7. Thiopyrane gemäß den Ansprüchen 1 oder 2 zur Verwendung bei der Bekämpfung von Kreislauferkrankungen.

8. Arzneimittel enthaltend mindestens ein Thiopyran gemäß Ansprüchen 1 oder 2.

9. Verfahren zur Herstellung von Arzneimitteln, dadurch gekennzeichnet, daß man Thiopyrane gemäß Ansprüchen 1 oder 2, gegebenenfalls unter Verwendung von üblichen Hilfs- und Trägerstoffen, in eine geeignete Applikationsform überführt.

10. Verwendung von Thiopyranen gemäß Ansprüchen 1 oder 2 für die Herstellung von Arzneimitteln zur Bekämpfung von Erkrankungen.

**Claims**

1. Thiopyrans of the general formula (I)

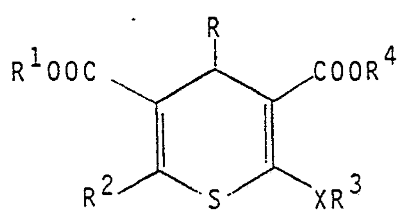

(I)

in which

R represents phenyl, naphthyl, furyl, pyrryl or pyridyl, these rings optionally containing 1, 2 or 3 identical or different substituents from the group comprising alkyl, alkenyl, alkinyl, alkoxy, alkylene, alkylenedioxy, alkylamino, each having up to 4 carbon atoms, phenyl, halogen, trifluoromethyl, trifluoromethoxy, nitro, cyano or azido,

$R^1$, $R^3$ and $R^4$ are identical or different and each represent hydrogen, an alkyl radical with up to 6 carbon atoms or one of the radicals mentioned under R, it being possible for $R^4$ also to represent a single bond to R with the formation of a 6- to 7-membered lactone ring ;

$R^2$ represents hydrogen, an alkyl radical with up to 4 carbon atoms, phenyl or aminoalkyl with up to 4 carbon atoms, and

X represents O, S or —NH—,

and their pharmaceutically acceptable acid addition salts.

2. Thiopyrans according to Claim 1, characterised in that in the general formula (I)

R represents phenyl, naphthyl or pyridyl, these rings optionally containing 1 or 2 identical or different substituents from the group comprising alkyl, alkoxy, alkylene, alkylenedioxy, alkylamino, each having up to 4 carbon atoms, phenyl, halogen, trifluoromethyl, trifluoromethoxy, nitro, cyano or azido,

$R^1$, $R^3$ and $R^4$ are identical or different and each represent hydrogen, an alkyl radical with up to 4 carbon atoms or phenyl, it being possible for $R^4$ also to represent a single bond to R with the formation of a 6- to 7-membered lactone ring,

$R^2$ represents hydrogen, an alkyl radical with up to 4 carbon atoms, phenyl or aminoalkyl with up to 4 carbon atoms, and

X represents O, S or —NH—,

and their pharmaceutically acceptable acid addition salts.

3. Process for the preparation of thiopyrans of the general formula (I)

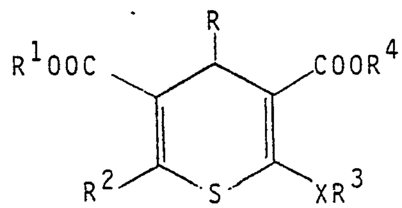

(I)

in which

R represents phenyl, naphthyl, furyl, pyrryl or pyridyl these rings optionally containing 1, 2 or 3 identical or different substituents from the group comprising alkyl, alkenyl, alkinyl, alkoxy, alkylene, alkylenedioxy, alkylamino, each having up to 4 carbon atoms, phenyl, halogen, trifluoromethyl, trifluoromethoxy, nitro, cyano or azido,

$R^1$, $R^3$ and $R^4$ are identical or different and each represent hydrogen, an alkyl radical with up to 6 carbon atoms or one of the radicals mentioned under R, it being possible for $R^4$ also to represent a single bond to R with the formation of a 6- to 7-membered lactone ring ;

$R^2$ represents hydrogen, an alkyl radical with up to 4 carbon atoms, phenyl or aminoalkyl with up to 4 carbon atoms, and

X represents O, S or —NH—,

and their pharmaceutically acceptable acid addition salts, characterised in that thio compounds of the general formula (II)

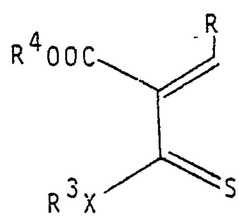

(II)

in which R, $R^3$, $R^4$ and X have the abovementioned meanings, are reacted with acetylene compounds of the general formula (III)

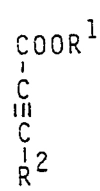

(III)

in which $R^1$ and $R^2$ have the abovementioned meanings, at temperatures between 0 °C and 200 °C, if appropriate in the presence of inert organic solvents.

4. Process according to Claim 3 for the preparation of thiopyrans of the general formula (I), in which

R represents phenyl, naphthyl, furyl, pyrryl or pyridyl, these rings optionally containing 1, 2 or 3 identical or different substituents from the group comprising alkyl, alkoxy, alkylene, alkylenedioxy, alkylamino, each having up to 4 carbon atoms, phenyl, halogen, trifluoromethyl, trifluoromethoxy, nitro, cyano or azido,

$R^1$, $R^3$ and $R^4$ are identical or different and each represent hydrogen, an alkyl radical with up to 4 carbon atoms or one of the radicals mentioned under R, it being possible for $R^4$ also to represent a single bond to R with the formation of a 6- to 7-membered lactone ring ;

$R^2$ represents hydrogen, an alkyl radical with up to 4 carbon atoms, phenyl or aminoalkyl with up to 4 carbon atoms, and

X represents O, S or —NH—,

and their pharmaceutically acceptable acid addition salts.

5. Process according to Claims 3 or 4, characterised in that the reaction is carried out at temperatures between 50 and 150 °C.

6. Process according to Claims 3 or 4, characterised in that the reaction is carried out in an excess of the acetylene carboxylic acid esters used or in an inert organic solvent such as toluene, ethanol, dioxan or ethylene glycol.

7. Thiopyrans according to Claims 1 or 2 for use in combating circulatory disorders.

8. Medicaments containing at least one thiopyran according to Claims 1 or 2.

9. Process for the preparation of medicaments, characterised in that thiopyrans according to Claims 1 or 2 are converted into a suitable form for administration, if appropriate using customary auxiliaries and excipients.

10. Use of thiopyrans according to Claims 1 or 2 for the preparation of medicaments for combating diseases.

**Revendications**

1. Thiopyrannes de formule générale (I)

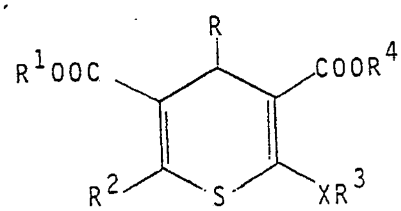

(I)

dans laquelle

R représente un noyau phényle, naphtyle, furyle, pyrryle ou pyridyle, ces noyaux comportant, le cas échéant, 1, 2 ou 3 substituants identiques ou différents choisis dans le groupe des radicaux alkyle, alcényle, alcynyle, alkoxy, alkylène, alkylènedioxy, alkylamino ayant chacun jusqu'à 4 atomes de carbone, phényle, halogéno, trifluorométhyle, trifluorométhoxy, nitro, cyano ou azido,

$R^1$, $R^3$ et $R^4$ sont identiques ou différents et représentent chacun l'hydrogène, un reste alkyle ayant jusqu'à 6 atomes de carbone ou l'un des restes mentionnés pour R, $R^4$ pouvant représenter en outre une liaison simple avec R, en formant un noyau de lactone hexagonal ou heptagonal,

$R^2$ représente l'hydrogène, un reste alkyle ayant jusqu'à 4 atomes de carbone, phényle ou aminoalkyle ayant jusqu'à 4 atomes de carbone, et

X représente O, S ou —NH—,

ainsi que leurs sels d'addition d'acides pharmaceutiquement acceptables.

2. Thiopyrannes suivant la revendication 1, caractérisés en ce que, dans la formule générale (I),

R désigne un noyau phényle, naphtyle ou pyridyle, ces noyaux comportant éventuellement 1 ou 2 substituants identiques ou différents choisis dans le groupe des substituants alkyle, alkoxy, alkylène, alkylènedioxy, alkylamino ayant chacun jusqu'à 4 atomes de carbone, phényle, halogéno, trifluorométhyle, trifluorométhoxy, nitro, cyano ou azido,

$R^1$, $R^3$ et $R^4$ sont identiques ou différents et représentent chacun l'hydrogène, un reste alkyle ayant jusqu'à 4 atomes de carbone ou le reste phényle, $R^4$ pouvant représenter en outre une liaison simple avec R, en formant un noyau de lactone hexagonal ou heptagonal,

$R^2$ est l'hydrogène, un reste alkyle ayant jusqu'à 4 atomes de carbone, phényle ou amino-alkyle ayant jusqu'à 4 atomes de carbone, et

X représente O, S ou —NH—,

ainsi que leurs sels d'addition d'acides pharmaceutiquement acceptables.

3. Procédé de production de thiopyrannes de formule générale (I)

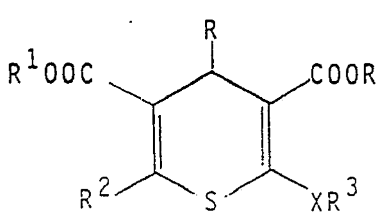

(I)

dans laquelle

R représente un noyau phényle, naphtyle, furyle, pyrryle ou pyridyle, ces noyaux comportant, le cas échéant, 1, 2 ou 3 substituants identiques ou différents choisis dans le groupe des radicaux alkyle, alcényle, alcynyle, alkoxy, alkylène, alkylènedioxy, alkylamino ayant chacun jusqu'à 4 atomes de carbone, phényle, halogéno, trifluorométhyle, trifluorométhoxy, nitro, cyano ou azido,

$R^1$, $R^3$ et $R^4$ sont identiques ou différents et représentent chacun l'hydrogène, un reste alkyle ayant jusqu'à 6 atomes de carbone ou l'un des restes mentionnés pour R, $R^4$ pouvant représenter en outre une liaison simple avec R, en formant un noyau de lactone hexagonal ou heptagonal,

$R^2$ représente l'hydrogène, un reste alkyle ayant jusqu'à 4 atomes de carbone, phényle ou aminoalkyle ayant jusqu'à 4 atomes de carbone, et

X représente O, S ou —NH—,

ainsi que de leurs sels d'addition d'acides pharmaceutiquement acceptables, caractérisé en ce qu'on fait réagir des composés thio de formule générale (II)

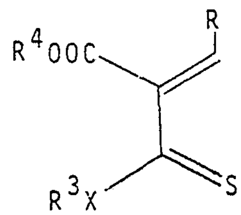

(II)

dans laquelle R, $R^3$, $R^4$ et X ont les définitions indiquées ci-dessus, à des températures comprises entre 0 et 200 °C, éventuellement en présence de solvants organiques inertes, avec des composés d'acétylène de formule générale (III)

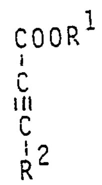

(III)

dans laquelle $R^1$ et $R^2$ ont les définitions indiquées ci-dessus.

4. Procédé suivant la revendication 3, pour l'obtention de thiopyrannes de formule générale (I), dans laquelle

R désigne un noyau phényle, naphtyle, furyle, pyrryle ou pyridyle, ces noyaux comportant éventuellement 1, 2 ou 3 substituants identiques ou différents choisis dans le groupe des substituants alkyle, alkoxy, alkylène, alkylènedioxy, alkylamino ayant chacun jusqu'à 4 atomes de carbone, phényle, halogéno, trifluorométhyle, trifluorométhoxy, nitro, cyano ou azido,

$R^1$, $R^3$ et $R^4$ sont identiques ou différents et représentent chacun l'hydrogène, un reste alkyle ayant jusqu'à 4 atomes de carbone ou l'un des restes mentionnés pour R, $R^4$ pouvant représenter en outre une liaison simple avec R, en formant un noyau de lactone hexagonal ou heptagonal,

$R^2$ est l'hydrogène, un reste alkyle ayant jusqu'à 4 atomes de carbone, phényle ou aminoalkyle ayant jusqu'à 4 atomes de carbone, et

X représente O, S ou —NH—

ainsi que de leurs sels d'addition d'acides pharmaceutiquement acceptables.

5. Procédé suivant la revendication 3 ou 4, caractérisé en ce qu'on conduit la réaction à des températures comprises entre 50 et 150 °C.

6. Procédé suivant la revendication 3 ou 4, caractérisé en ce qu'on conduit la réaction dans un excès des esters d'acides acétylènecarboxyliques utilisés ou dans un solvant organique inerte tel que le toluène, l'éthanol, le dioxanne ou l'éthylène-glycol.

7. Thiopyrannes suivant la revendication 1 ou 2, destinés à être utilisés pour combattre des maladies du système circulatoire.

8. Médicaments contenant au moins un thiopyranne suivant la revendication 1 ou 2.

9. Procédé de préparation de médicaments, caractérisé en ce que des thiopyrannes suivant la revendication 1 ou 2 sont mis sous forme administrable appropriée en utilisant éventuellement des adjuvants et des supports classiques.

10. Utilisation de thiopyrannes suivant la revendication 1 ou 2 pour la préparation de médicaments destinés à combattre des maladies.